# EUROPÄISCHE PATENTSCHRIFT

(11) **EP 2 386 309 B1**
(45) Veröffentlichungstag und Bekanntmachung des Hinweises auf die Patenterteilung: **12.08.2015**
(21) Anmeldenummer: 11165713.6
(22) Anmeldetag: 11.05.2011
(51) Int. Cl.: A61Q 19/00, A61K 8/97, A61K 47/10, A61K 36/88, A61K 9/00

(54) **Bulbine frutescens Gel**
Bulbine frutescens gel
Bulbine frutescens gel

(30) Priorität: 12.05.2010 DE 102010028960
(43) Veröffentlichungstag der Anmeldung: 16.11.2011
(73) Patentinhaber: Botanica Natural Products (Pty) Ltd, Alldays, Limpopo 0909 (ZA)
(72) Erfinder: Colson, Michel Henri., 75015 Paris (FR)
(74) Vertreter: RGTH

(56) Entgegenhaltungen:
- US-A- 3 892 853
- US-A- 4 178 372
- MOTSEI M L ET AL: "Screening of traditionally used South African plants for antifungal activity against Candida albicans", JOURNAL OF ETHNOPHARMACOLOGY 200306 IE LNKD- DOI:10.1016/S0378-8741(03)00082-5, Bd. 86, Nr. 2-3, Juni 2003 (2003-06), Seiten 235-241, XP55004288, ISSN: 0378-8741
- PATHER N ET AL: "A biochemical comparison of the in vivo effects of Bulbine frutescens and Bulbine natalensis on cutaneous wound healing", JOURNAL OF ETHNOPHARMACOLOGY, ELSEVIER SCIENTIFIC PUBLISHERS LTD, IE, Bd. 133, Nr. 2, 27. Januar 2011 (2011-01-27), Seiten 364-370, XP027596112, ISSN: 0378-8741, DOI: 10.1016/J.JEP.2010.10.007 [gefunden am 2011-01-11]
- COOPOOSAMY R M: "Traditional information and antibacterial activity of four Bulbine species (Wolf)", AFRICAN JOURNAL OF BIOTECHNOLOGY, ACADEMIC PRESS, US, Bd. 10, Nr. 2, 10. Januar 2011 (2011-01-10), Seiten 220-224, XP009151045, ISSN: 1684-5315
- WIDGEROW A., ET AL.: "New Innovations in Scar Management", AESTH. PLAST. SURG., vol. 24, 2000, pages 227-234, DOI: 10.1007/s002660010038
- KIEPENKERL: "Duftgeranien aus Suedafrika", GARTENWELT AKTUELL, May 2010 (2010-05),
- JOHN MITCHELL WATT, MARIA GERDINA BREYER-BRANDWIJK: "The Medicinal and Poisonous Plants of Southern and Eastern Africa", 1962, LIVINGSTONE, EDINBURGH, LONDON pages 695-696,

## Beschreibung

Die Erfindung betrifft ein Bulbine-frutescens-Gel, ein Verfahren zu dessen Herstellung, dessen Verwendung im medizinischen und kosmetischen Bereich sowie Zusammensetzungen, umfassend das erfindungsgemäße Gel.

Die Bulbine frutescens (L.), die zur Familie der Asphodeloideae (Affodillgewächse) gehört, ist auch unter den Bezeichnungen "Rankkopieva", "lbhucu" oder "Ithethe elimpofu" bekannt. Der deutsche Name lautet Stelzen-Bulbine. Die Pflanze ist in ganz Südafrika weit verbreitet; wobei mehr als 40 Unterarten bekannt sind. Sie wächst bevorzugt in sandigen Böden. Die Pflanze hat einen Wurzelstock mit zahlreichen drahtartigen Wurzeln, die Blätter sind 15 cm lang und 4 bis 8 mm dick, von hellgrüner Farbe, unbehaart und fleischig. Von April bis August trägt die Pflanze gelbe, orangefarbene oder weiße Blüten in dichten länglichen Blütentrauben mit einer Länge bis zu 30 cm und bärtigen Staubblättern.

Bekannt ist es, durch Auspressen aus den Blättern einen geleeartiger Saft zu gewinnen, der als "Allheilmittel" beispielsweise gegen (brennende) Wunden, Schürfwunden, wunde Stellen, Hautausschlag, Ekzeme und Ringelflechte verwendet wird. Der Saft wird aus einem frisch gepflückten Blatt gepreßt und auf den betroffenen Hautbereich aufgetragen. Es ist außerdem ein Tee-Aufguss aus den frischen Blätter bekannt, der gegen Erkältung, Husten, Arthritis, Durchfall, Blasenentzündungen, Infektionen der Harnwege und bei einigen sexuell übertragbaren Krankheiten helfen soll. Die Wirksamkeit ist bisher nicht klinisch erwiesen und es sind auch keinerlei Fachberichte hierüber veröffentlicht worden.

Zur chemischen Zusammensetzung sind jedoch einige Informationen verfügbar. So berichtet Van Staden (Phytochemistry 35, 685 (1994)), dass Knipholon in Bulbine latifolia und Bulbine frutescens vorhanden ist. Knipholon ist abgeleitet von 1,8-Dihydroxyanthrachinon und trägt in der 3-Stellung eine Methylgruppe und in der 4-Stellung eine 2,6-Dihydroxy-4-methoxy-3-acetylphenylgruppe, die auch als 2-Acetylphloroglucinmethylether bezeichnet wird; Isoknipholon weist die 2-Acetylphloroglucinmethylethergruppe in der 9-Stellung auf. 1,8-Dihydroxy-3-methylanthrachinon ist besser bekannt als Chrysophanol.

Das Vorkommen von Knipholon und seinen Derivaten ist praktisch auf die Bulbinearten beschränkt, obwohl Anthrachinone in der Natur weit verbreitet sind. Knipholon wurde nur in Kniphofia-Arten gefunden, wie beispielsweise von Dagne und Steglich (Phytochemistry 23, 1729 (1984) und Bull. Chem. Soc. Ethiop. 32, 1 (1987)) beschrieben. Leistner et al. (Chem. Pharm. Bull. 52, 1262 (2004)) haben Knipholon-Derivate in Kniphofia foliosa und Kniphofia tuckii, die in Äthiopien beheimatet sind, mit Schwerpunkt auf der Glucose und dem Disaccharid Gentiobiose als glucosidischem Teil dieser wasserlöslichen Anthrachinone beschrieben.

Seit dem ersten Bericht über Knipholon in Vertretern der Familie der Asphodeloideae wurde über eine Anzahl weiterer arylsubstituierter Chrysophanol-Derivate, entweder als Glycoside oder als Aglycone, berichtet. Bringmann et al. (J. Nat. Prod. 65, 1117 (2002)) beschrieben Gaborochinon A und B, 4'-*O*-Demethylknipholon-4'-*O*-β-d-glucopyranosid und Phenylanthrachinone aus den Wurzeln der Bulbine frutescens. Einige dieser Verbindungen zeigten *in vitro* moderate bis gute antiplasmodiale und antitrypanosomale Wirkungen. Bringmann et al. (2007) fanden außerdem in den Wurzeln der Bulbine frutescens dimere Knipholon-Derivate (Joziknipholon A und B). *In vitro* zeigten diese Joziknipholone Wirkung gegen den chloroquinresistenten Strang K1 des Malariaerregers Plasmodium falciparum und moderate Wirkung gegen L5178Y-Leukämie-Lymphom-Mäusezellen.

Rabe und Van Staden (J. Ethnopharmacology 56, 81 (1997)) untersuchten die In-vitro-Wirkung getrockneter Extrakte aus den Blättern der Bulbine frutescens auf ihre mikrobiologische Wirkung gegen Staphylococcus aureus, Staphylococcus epidermidis and Bacillis subtilis; die Extrakte erwiesen sich jedoch als unwirksam. Van Staden und Drewes testeten Knipholon *in vitro* auf antimikrobielle Wirkung gegen Escherichia coli, Pseudomonas aeruginosa, Staphylococcus aureus, Bacillus subtilis, Micrococcus luteus und Candida albicans; dieses erwies sich jedoch ebenfalls als nicht wirksam. Berichte zur Bioverfügbarkeit von Bulbine frutescens wurden bislang nicht veröffentlicht.

Substituierte 1,8-Dihydroxyanthrachinone sind auch in verschiedenen Aloearten, wie Aloe barbadensis, Aloe ferox (welche ebenfalls zur Familie der Asphodeloideae gehören) und in Rhabarberarten, wie Rheum rhabarbarum und Rheum palmatum (Chinesischer Rhabarber), zu finden. Den Anthrachinonen in den Aloe- und Rhabarberarten fehlt die in Knipholon vorhandene 2-Acetylphloroglucinmethylethergruppe; die Methylgruppe in der 3-Stellung kann auch in oxidierter Form vorhanden sein, entweder als Hydroxymethylgruppe, als Aldehyd oder als Säure. Die Anthrachinone in den Aloe- und Rhabarberarten sind als Glycoside vorhanden; die Zuckerreste können Pentosen und/oder Hexosen sein. Diese sind gut wasserlöslich, aber nach Alterung dieser Extrakte oder übermäßiger Erhitzung kann Hydrolyse auftreten und die Anthrachinone werden in Wasser unlöslich.

Die in Bulbinearten vorhandenen Anthrachinone zeigen ein völlig anderes physiologisches Potential als die Anthrachinone, die aus Aloe- und Rhabarberarten stammen. Die Anthrachinone aus Aloe- und Rhabarberarten zeigen oft abführende Eigenschaften. Die grundlegende Verbindung 1,8-Dihydroxyanthrachinon (Danthron®) wurde 1987 weltweit aus dem Handel genommen, da begründeter Verdacht bestand, dass diese Verbindung für den Menschen karzinogen ist. Toxische Nebenwirkungen, wie bei Aloe-/Rhabarber-Anthrachinonen, treten bei Knipholon-Anthrachinonen im Wesentlichen jedoch nicht auf. Hersteller von Aloegelen sind demnach stark bemüht, den Anthrachinon-Anteil so weit wie möglich zu reduzieren, da bei Aloegelen hauptsächlich der Polysaccharidanteil von Bedeutung ist.

Weiterhin ist aus der US 2010/0062085 A1 von A. D. Widgerow sowie aus weiteren US-Patentschriften von Widgerow und Chait bekannt, aus einem Extrakt der Bulbine frutescens ein Gel in Kombination mit Centella asiatica (auch bekannt als Tigergras, Indischer Wassernabel oder Gotu Cola) und Panthenol, der Vorstufe von Pantothensäure, zur Behandlung von Narben und Akne einzusetzen. Centella asiatica ist eine bekannte Quelle für eine Vielzahl pentacyclischer Triterpenoide, wie Asiaticosid, Centellosid und Madecassosid, nebst zahlreichen anderen chemischen Verbindungen. Die Offenbarung der US 2010/0062085 A1 erscheint jedoch äußerst fragwürdig, da der Beitrag von Bulbine frutescens bei der Verhinderung der Vernarbung klinisch nicht nachgewiesen ist.

Motsei, M. L. et al ("Screening of traditionally used South African plants for antifungal activity against Candida albicans", Journal of Ethnopharmacology 86 (2003), Seiten 235 bis 241) prüften 24 südafrikanische medizinische Pflanzen gegen den Stamm ATTC 10231 des Candida albicans zur Erlangung eines Heilmittels gegen orale Candidiasis, welche insbesondere bei HIV-Patienten verbreitet ist. Unter anderem wurden in diesem Zusammenhang auch Bulbine Frutescens-Blätter getestet.

Pather, N. et al. ("A biochemical comparison of the in vivo effects of Bulbine frutescens and Bulbine natalensis on cutaneous wound healing"; Journal of Ethnopharmacology, Band 133, Nr. 2, 27. Januar 2011, Seiten 364 bis 370) offenbaren, dass Bulbine Frutescens nur in Südafrika einheimisch und weit verbreitet als Heilmittel für die Haut ist. In dem Artikel untersuchen Pather, N. et al. den wissenschaftlichen Wert dieser Pflanze mittels Überprüfung der in vivo biochemischen Effekte von Bulbine Frutescens auf Hautwunden.

Coopoosamy, R. M. ("Traditional information and antibacterial activity of four Bulbine species (Wolf); African Journal of Biotechnology, Band 10, Nr. 2, 10. Januar 2011, Seiten 220 bis 224) offenbart, dass Bulbine Frutescens die in Südafrika am weitesten verbreitetste benutzte Bulbineart ist und insbesondere für die Behandlung von Durchfall, Verbrennungen, Hautausschlägen, Blasen, Insektenstichen, aufgeplatzten Lippen und Mundgeschwüren gesammelt wird.

Die US 3,892,853 A betrifft ein Verfahren zur Stabilisierung eines klaren Gels von Aloeverablättern, um eine haltbare Zubereitung zu erreichen, die die therapeutischen Eigenschaften des frischen Gels beibehält.

Die US 4,178,372 A betrifft ebenfalls ein Verfahren zur Stabilisierung eines klaren Gels von Aloeverablättern.

Es besteht demnach ein Bedarf weitere Mittel für unterschiedliche therapeutische und andere Zweck bereitzustellen, die gegenüber bekannten Verbindungen über ein verbessertes und nachweisbares Wirkungsspektrum verfügen, in verschiedenen Bereichen zum Einsatz kommen können, in einfacher Weise erhältlich und anwendbar sind.

Die vorliegende Erfindung betrifft ein Bulbine-frutescens-Gel, ein Verfahren zu dessen Herstellung sowie die Verwendung des Bulbine-frutescens-Gels im medizinischen oder kosmetischen Bereich. Gegenstand der Erfindung ist auch eine pharmazeutische oder kosmetische Zusammensetzung, umfassend das Gel. Die Verwendung im medizinischen Bereich betrifft im Wesentlichen die Behandlung von Haut- oder Schleimhauterkrankungen, insbesondere solche mit mikrobiologischer Ursache, beispielsweise hervorgerufen durch opportunistische Mikroorganismen oder durch hypo-/hyperaktive Enzyme. Im kosmetischen Bereich kann das Gel beispielsweise zur Körperpflege eingesetzt werden. Das Gel der Erfindung dient auch zur Stärkung des Immunsystems und ist daher zur Behandlung von immungeschwächten Personen besonders gut geeignet.

Die Erfindung bezieht sich daher auf ein Bulbine-frutescens-Gel, erhältlich durch die Schritte:
(1) Waschen der Blätter der Bulbine frutescens mit Wasser und gegebenenfalls Zerkleinern der Blätter;
(2) Auspressen der Blätter unter Erhalt eines gelartigen Safts;
(3) Behandeln des gelartigen Safts mit Wasserstoffperoxid unter Rühren, wobei pro kg Saft 0,5 bis 20 g Wasserstoffperoxid zugegeben werden;
(4) Zerstören des Wasserstoffperoxids durch Zugabe einer pharmazeutisch oder kosmetisch akzeptablen Säure unter Rühren bis der Peroxidwert unter 0,02 meqO/kg liegt;
(5) Einstellen des pH-Werts auf 3,0 bis 4,0 unter Zugabe einer pharmazeutisch oder kosmetisch akzeptablen Säure und
(6) Erhalt des Gels unter Rühren sobald alle Komponenten gelöst sind.

Nachfolgend werden die einzelnen Verfahrensschritte des erfindungsgemäßen Verfahrens zur Gewinnung des Bulbine-frutescens-Gels detailliert beschrieben:
Die Blätter der Bulbine frutescens können das ganze Jahr hindurch geerntet werden, wobei in der Regel mindestens zwei Mal geerntet wird. Es ist zweckmäßig, die Blätter direkt nach dem Ernten schnell weiterzuverarbeiten. Nach dem Ernten werden die Blätter zunächst mit Wasser gewaschen (Verfahrensschritt (1)), um Erde und Schmutz zu entfernen. Der gelartige Saft wird mit Hilfe geeigneter Vorrichtungen aus den Blättern heraus gepreßt (Verfahrensschritt (2)). Gegebenenfalls können die Blätter in kleine Stücke geschnitten werden, um das Auspressen zu erleichtern. Der gewonnene gelartige Saft ist nahezu farblos, mit einem leicht gelblichen Farbton, und ist einem Gel ähnlich, d.h. viskos oder leicht viskos, wobei die Viskosität u.a. vom Zeitpunkt der Ernte und vom Alter der Pflanze abhängt.

Der gewonnene Saft wird anschließend mit Wasserstoffperoxid behandelt (Verfahrensschritt (3)), während gerührt wird. Vorteilhafterweise kann hierfür ein Planetenmischer eingesetzt werden. Es können aber auch beliebige andere Rührer verwendet werden. Eine gute Durchmischung sollte in jedem Fall gewährleistet werden.

Der Saft wird mit einer derartigen Menge an Wasserstoffperoxid versetzt, dass pro Kilogramm Saft 0,5 bis 20 g Wasserstoffperoxid verwendet werden, berechnet als H₂O₂. Die genaue Menge hängt zum Beispiel vom Zeitpunkt der Ernte und dem Alter der geernteten Pflanzen ab. Das Wasserstoffperoxid wird im erfindungsgemäßen Verfahren eingesetzt, um die Gelstruktur zu zerstören, die von verzweigten Polysacchariden herrührt, und außerdem, um die vorhandenen Enzyme zu denaturieren. Es wurde beobachtet, dass die Wirkung von Enzymresten nachteilig ist, da diese zu einem Verlust der Wirkung beim erhaltenen Gel-Endprodukt, zu verstärkter Farbbildung und zur Glycolyse der Knipholonglycoside und deren Derivaten führen. Die Anwendungszeit des Wasserstoffperoxids ist im Rahmen der Erfindung nicht besonders beschränkt, es ist jedoch vorteilhaft, die Anwendungszeit so kurz wie möglich einzustellen, um oxidative Nebenreaktionen zu vermeiden, die zu einer Verfärbung führen würden. Bevorzugt wird die Behandlungsdauer im Verfahrensschritt (3) daher derart gewählt, dass keine Verfärbung resultiert. Bevorzugt ist eine Anwendungsdauer von wenigen Minuten bis zu 1 Stunde, besonders bevorzugt von wenigen Minuten bis etwa eine halbe Stunde. Ganz besonders bevorzugt beträgt die Behandlungsdauer 5 bis 20 Minuten. Während des Verfahrens können einige, bisher noch nicht identifizierte Oxidationsprozesse auftreten, die zu unerwünschten Verfärbungen und zur Glycolyse der Knipholonglycoside führen. Diese können durch genaue Kontrolle und möglichst kurzen Behandlungszeitraum auf ein Mindestmaß reduziert werden.

Im sich anschließenden Verfahrensschritt (4) wird verbliebenes Wasserstoffperoxid durch die Zugabe von ein oder mehreren pharmazeutisch oder kosmetisch akzeptablen Säuren zerstört. Der Begriff "pharmazeutisch oder kosmetisch akzeptabel" bedeutet im Rahmen der vorliegenden Erfindung Verbindungen, welche für die beschriebene Behandlung eingesetzt werden können und aus medizinischer oder kosmetischer Sicht nicht unerwünscht sind, die im Rahmen einer medizinischen oder kosmetischen Verabreichung an einen Verwender bei Kontakt mit der Haut- oder Schleimhaut keinen nachteiligen und/oder schädlichen Einfluß zeigen, beispielsweise keine Reizungen, Allergien oder andere Probleme beim Verwender in Anbetracht der Schwere der Beeinträchtigung oder Erkrankung und der Notwendigkeit der Behandlung auslösen. Pharmazeutisch oder kosmetisch akzeptable Säuren sind beispielsweise ausgewählt aus organischen Säuren. Beispielhaft seien genannt: Adipinsäure, Alginsäure, Ascorbinsäure, Isoascorbinsäure (Erythorbinsäure), Asparaginsäure, Benzolsulfonsäure, Bernsteinsäure, 2-Acetoxybenzoesäure, Camphersäure, Camphersulfonsäure, Zimtsäure, Citronensäure, insbesondere Citronensäure-Monohydrat, Digluconsäure, Ethansulfonsäure, Glutaminsäure, Glykolsäure, Heptansäure, Hexansäure, Fumarsäure, 2-Hydroxyethansulfonsäure (Isethionsäure), Milchsäure, Maleinsäure, Hydroxymaleinsäure, Äpfelsäure, Malonsäure, Mandelsäure, Pamoasäure, Pectinsäure, Phenylessigsäure, 3-Phenylpropionsäure, Picrinsäure, Pivalinsäure, Pyruvinsäure, Stearinsäure, Sulfanilsäure, Weinsäure, p-Toluolsulfonsäure und dergleichen.

Besonders bevorzugt werden in Verfahrensschritt (4) Ascorbinsäure und insbesondere bevorzugt Isoascorbinsäure (Erythorbinsäure) verwendet. Selbstverständlich können auch Mischungen von pharmazeutisch oder kosmetisch akzeptablen Säuren zum Einsatz kommen.

Nach der Behandlung mit der pharmazeutisch oder kosmetisch akzeptablen Säure, wie beispielsweise Ascorbinsäure oder Isoascorbinsäure, sollte der Peroxidwert unter 0,02 meqO/kg liegen.

Anschließend wird in Verfahrensschritt (5) des erfindungsgemäßen Verfahrens mit Hilfe einer pharmazeutisch oder kosmetisch akzeptablen Säure der pH-Wert auf 3,0 bis 4,0 eingestellt. Hierzu kann eine der oben beschriebenen Säuren eingesetzt werden. Besonders bevorzugt wird in Verfahrensschritt (5) Citronensäure-Monohydrat verwendet.

In Schritt (6) wird das Gel-Endprodukt durch Rühren erhalten, sobald alle Komponenten vollständig gelöst sind und keine Aggregate mehr vorhanden sind. Das Rühren kann mit einer beliebigen Rührvorrichtung durchgeführt werden. Besonders bevorzugt ist ein Planetenrührer.

Der mikrobiologische Zustand und das Vorhandensein von pathogenen Organismen im erhaltenen Gel der Erfindung werden entsprechend den in verschiedenen Arzneibüchern beschriebenen Methoden, wie dem Europäischen Pharmakopöe und dem United States Pharmacopoeia, kontrolliert. Die aerobe Keimzahl liegt bevorzugt bei weniger als 100 CFU/g, vorzugsweise bei maximal 10 CFU/g, und es sollten im Wesentlichen keine Pathogene vorhanden sein. Es versteht sich, dass die gewünschte Keimfreiheit und Freiheit von Pathogenen durch entsprechendes Arbeiten unter sterilen Bedingungen mit sterilen Arbeitsgeräten und entsprechend kontrollierten Ausgangsstoffen erreicht werden kann.

Es ist anzumerken, dass es zweckmäßig ist, das gewonnene Bulbine-frutescens-Gel gegen opportunistische Mikroorganismen ("Mitläufer") zu schützen. Govender, du Plessis-Stoman, Downing und Van De Venter (South African Journal of Science, 102, 253 (2006)) kamen zu dem Schluss, dass Produkte, die auf lokalen Märkten angeboten werden, mikrobiologisch kontaminiert waren und als Gesundheitsrisiko für den Menschen zu betrachten sind.

Vorteilhafterweise werden daher dem erfindungsgemäßen Gel ein oder mehrere pharmazeutisch oder kosmetisch akzeptable Konservierungsmittel zugesetzt. Der Begriff "pharmazeutisch oder kosmetisch akzeptabel" bedeutet im Rahmen der vorliegenden Erfindung Verbindungen, welche für die beschriebene Behandlung eingesetzt werden können und aus medizinischer oder kosmetischer Sicht nicht unerwünscht sind, die im Rahmen einer medizinischen oder kosmetischen Verabreichung an einen Verwender bei Kontakt mit der Haut- oder Schleimhaut keinen nachteiligen und/oder schädlichen Einfluß zeigen, beispielsweise keine Reizungen, Allergien oder andere Probleme beim Verwender in Anbetracht der Schwere der Beeinträchtigung oder Erkrankung und der Notwendigkeit der Behandlung auslösen. Pharmazeutisch oder kosmetisch akzeptable Konservierungsmittel sind beispielsweise: Benzoesäure, Sorbinsäure, Parabenen (Methyl-, Ethyl-, Propyl-, Butyl-, Isobutyl- oder Benzyl-Paraben), Ameisensäure, Essigsäure, Propionsäure, Salicylsäure, p-Anissäure, Lävulinsäure und deren wasserlöslichen Salzen, Glycerol-/Polyglycerolmonoestern von Caprylsäure, Caprinsäure-Laurinestern, speziellen Diolen, wie 1,2-Dihydroxypentan, 1,2-Dihydroxyhexan oder 1,2-Dihydroxyoctan, oder 1-(2-Ethylhexyl)-Glycerylether und dergleichen. Es können auch Mischungen verwendet werden.

Das erfindungsgemäß gewonnene Bulbine-frutescens-Gel kann so verwendet werden, wie zuvor beschrieben hergestellt, oder es kann erst nach einer Weiterverarbeitung eingesetzt werden. Die Weiterverarbeitung kann ausgewählt sein aus ein oder mehreren der nachfolgenden Verfahrensweisen, die auch in beliebiger Reihenfolge kombiniert werden können: Filtrieren, Wasserverdampfung unter vermindertem Druck / erhöhter Temperatur, azeotrope Destillation unter vermindertem Druck / erhöhter Temperatur unter Verwendung eines geeigneten Co-Lösemittels, Sprühtrocknen oder Gefriertrocknen. Der Nachteil von Verfahren unter erhöhter Temperatur besteht in dem Risiko der Umwandlung der Anthrachinonglycoside in die entsprechenden Aglykone, die in Wasser schwer löslich / unlöslich sind und so die Wirkung des Bulbine-frutescens-Gels vermindern. Die Gefriertrocknung hat den Vorteil, dass die Umwandlung der Anthrachinonglycoside in die entsprechenden Aglykone stark gehemmt wird. Das Vorhandensein von Aglykonen kann beispielsweise durch Lösen des unlöslichen Teils in Aceton/DMSO mit nachfolgender Zugabe von Kaliumhydroxid getestet werden. Es zeigt sich eine intensive Rot-/Magenta-/Violettfärbung.

Gegenstand der vorliegenden Erfindung ist auch die Verwendung des erfindungsgemäßen Bulbine-frutescens-Gels im therapeutischen oder kosmetischen Bereich.

Die Verwendung im kosmetischen Bereich umfasst die Körper- und Schönheitspflege, d.h. die Erhaltung, Wiederherstellung oder Verbesserung der Schönheit des menschlichen Körpers, beispielsweise durch Reinigen, Stabilisieren, Vitalisieren und/oder Deodorieren der Haut und Reinigen, Stabilisieren und/oder Vitalisieren der Schleimhaut.

Die Verwendung im therapeutischen Bereich umfasst insbesondere die Anwendung des Bulbine-frutescens-Gels zur Behandlung oder Vorbeugung von Krankheiten und Verletzungen der Haut und Schleimhaut. Auch Erkrankungen der Hautanhangsgebilde, wie Haare, Nägel, Talg- und Schweißdrüsen, werden zu den Hautkrankheiten gezählt, da diese auch von epidermaler Abstammung sind. Bekanntermaßen verweisen die bisherigen Veröffentlichungen in der Literatur darauf, dass im Wesentlichen keine antimikrobiellen Eigenschaften der Bulbine frutescens vorhanden sein sollen. In überraschender Weise wurde nun in der vorliegenden Erfindung festgestellt, dass das erfindungsgemäße Bulbine-frutescens-Gel über unerwartete antimikrobielle Eigenschaften verfügt. Die Versuche, die nachfolgend im experimentellen Teil beschrieben sind, zeigten Ergebnisse, die äußerst überraschend, völlig unerwartet und im Widerspruch zu früheren, in der Literatur angegebenen Daten sind. Die nachgewiesenen antimikrobiellen Eigenschaften des erfindungsgemäßen Bulbine-frutescens-Gels ermöglichen die Bereitstellung von Produkten mit Vorteilen im Hinblick auf besondere antimikrobielle Anforderungen. Die Versuche zeigten auch, dass das Bulbine-frutescens-Gel zwar kein generell wirksames antimikrobielles Mittel darstellt, da die völlige Abtötung bestimmter Organismen nicht stets erreicht wird, aber es stellt einen funktionellen Inhaltsstoff dar, der eine unerwartete antimikrobielle Wirkung in bestimmtem Maße bereitstellt.

Weiter Versuche haben gezeigt, dass sich Bulbine-frutescens-Gel, angereichert mit einer kleinen Menge Wasserstoffperoxid (z.B. 0,05 Gew.-%, ausgedrückt als H₂O₂), gegen bestimmte Organismen wie ein Desinfektionsmittel verhält, insbesondere gegen Staphylococcus aureus, einschließlich Methicillin-resistenten Staphylococcus aureus (MRSA) und Clindamycin-, Erythromycin- und Ciprofloxacin-resistenten Staphylococcus aureus (CEC-SA). Diese resistenten Stränge sind für gewöhnlich schwer zu kontrollieren, insbesondere in einer Krankenhausumgebung. Die Tests haben außerdem gezeigt, dass viele andere Organismen durch die beschriebene Zusammensetzung nicht oder nur eingeschränkt beeinflusst werden, insbesondere die natürliche Mikroflora der Haut und der Schleimhäute, wodurch die natürliche Mikroflora der Haut und der Schleimhäute geschont werden kann. Daher eignet sich Bulbine-frutescens-Gel, insbesondere angereichert mit einer kleinen Menge Wasserstoffperoxid (z.B. 0,05 Gew.-%, ausgedrückt als H₂O₂), insbesondere zur Desinfektion von Operationssälen.

Vergleichsversuche, bei denen Wasserstoffperoxid (z.B. 0,05 Gew.-%, ausgedrückt als H₂O₂) allein getestet wurde, erbrachten im Gegensatz zu den Versuchen von Bulbine-frutescens-Gel, angereichert mit Wasserstoffperoxid (z.B. 0,05 Gew.-%, ausgedrückt als H₂O₂), nicht die abtötenden Eigenschaften. Es wird daher angenommen, dass die Zugabe von Wasserstoffperoxid in kleinen Mengen im Bereich von >0 Gew.-% bis < 0,1 Gew.-% H₂O₂, zum Bulbine-frutescens-Gel eine synergistische Wirkung hat, da diese die Summe der beiden Einzelwirkungen deutlich übersteigt.

Das Bulbine-frutescens-Gel besteht aus vielen verschiedenen chemischen Einheiten, und dazu gehören auch Polysaccharide. Diese haben sich als nützlich bei der Wundheilung erwiesen, wie von Pather (Thesis, 2009) demonstriert. Es wurden daher In-vitro-Studien durchgeführt, um die Zytotoxizitäts- und Zellproliferationswirkungen des Bulbine-frutescens-Gels zu bestimmen. Das Gel weist keine zytotoxischen Eigenschaften auf und die Zellteilung war besser als 100 % bei 0,1 µg/ml. Weiterhin wurde festgestellt, dass das Bulbine-frutescens-Gel eine besonders starke wundheilende Wirkung bei Verletzungen der Haut- und Schleimhaut aufweist. Biochemische Analysen von behandeltem Wundgewebe zeigten im Vergleich zu unbehandelten Wunden eine deutlich erhöhte Menge Collagen, Einweiß und DNA-Gesamtgehalt. Ausgeschnittene und eingeschnittene Wunden in Schweinehaut zeigten im Vergleich zu unbehandelten Wunden eine stark erhöhte Geschwindigkeit beim Wundverschluss und eine vollständige Reepithelialisierung. Eine gute Wundheilung ist wichtig, da klinische Infektionen auftreten können, wenn die Bakterienkolonien in und um die Wundstellen auf ein kritisches Maß anwachsen. Dies kann zu einer Verschlechterung des Wundzustandes und, wenn systemische Infektionen auftreten, für den Patienten zu grippeähnlichen Symptomen mit Fieber führen. Häufig sind systemische Antibiotika erforderlich und wesentlicher Bestandteil der Behandlung. Bei frühzeitiger Behandlung mit dem Bulbine-frutescens-Gel kann aufgrund eines günstigen Verlaufs bei der Wundheilung daher auf die Verabreichung von Antibiotika verzichtet werden.

Das Bulbine-frutescens-Gel kann auch vorbeugend zur Vermeidung von Wunden, beispielsweise Brandwunden, oder zur schnellen Heilung von (Brand- )Wunden eingesetzt werden. Das Bulbine-frutescens-Gel ist außerdem besonders erfolgreich bei der Wundheilung kleiner Wunden anwendbar, die beispielsweise bei der Rasur und der Entfernung von Haaren und von einwachsenden Haaren bei Männern mit stark gekraustem Haar entstehen, bei schlecht heilenden Wunden, die beispielsweise durch eine Infektion durch Methicillin-resistenten Staphylococcus aureus (MRSA) und Clindamycin-, Erythromycin- und Ciprofloxacin-resistenten Staphylococcus aureus (CEC-SA) (besonders an den Füßen) entstanden sind, und auch bei anderen kleinen Wunden.

Das Bulbine-frutescens-Gel hat sich außerdem bei der Behandlung von Furunkeln und Karbunkeln bewährt.

Weiterhin wird angenommen, dass Arabinogalactane (AG's) die wichtigsten Polysaccharide sind. Diese Polysaccharide sind aus Arabinose und Galactose (Monosaccharide) zusammengesetzt; Arabinogalactane werden von Pflanzen erzeugt. Arabinogalactane sind zum Beispiel in großen Mengen in Gummi arabicum und Gummi ghatti zu finden. Außerdem erzeugen bestimmte Mikroorganismen Arabinogalactane. An Arabinogalactan angehängte Proteine (AGP) dienen als Signalmoleküle zwischen den Zellen sowie als Kleber zum Verschließen von Wunden an Pflanzen. Arabinogalactane sind dafür bekannt, das Immunsystem von Pflanzen zu stimulieren, da sie die Aktivität natürlicher Killerzellen und anderer Komponenten des Immunsystems steigern (A. M. Showalter, Cell. Mol. Life Sci., 58, 1399 (2001)). Obwohl dies besonders für Pflanzen gilt, können sie eventuell auch den menschlichen Körper beim Kampf gegen Infektionen unterstützen, wie von Yamada & Kiyohara ("Complement-activating polysaccharides from medicinal herbs", Immuno-modulatory Agents from Plants, 161- 202 (1999), H. Wagner (Hg.), Birkhäuser Basel) vorgeschlagen.

Durch Versuche konnte nun gezeigt werden, dass das Bulbine-frutescens-Gel tatsächlich hochgradig immunstärkende Eigenschaften aufweist. Dies belegen Versuche, bei denen das Bulbine-frutescens-Gel bei der Wundheilung immungeschwächter Personen getestet wurde, insbesondere bei Patienten mit Diabetes Typ II mit schlechter Wundheilung an den unteren Extremitäten und Füßen. Auch bei Wunden, die bereits seit längerer Zeit bestanden, konnte mit dem erfindungsgemäßen Gel eine Wundheilung erreicht werden, wenn auch zum Teil unter Ausbildung einer Narbe. Das Bulbine-frutescens-Gel kann daher auch bei der Wundheilung schlecht heilender Wunden, wie etwa bei Patienten mit Diabetes Typ II oder AIDS-Patienten, erfolgreich eingesetzt werden.

Gegenstand der vorliegenden Erfindung ist daher auch die Verwendung des Bulbine-frutescens-Gels als Mittel zur Stärkung der Immunabwehr.

Durch die Kombinationswirkung des Bulbine-frutescens-Gels, das neben der heilenden Wirkung auch über immunstärkende Eigenschaften verfügt, wird das Bulbine-frutescens-Gel daher zur Vermeidung/Vorbeugung und schnellen Heilung besonders erfolgreich eingesetzt.

Das erfindungsgemäße Bulbine-fructescens-Gel kann auch wirksam zur Bekämpfung von Nagel- und Fußpilz eingesetzt werden. Nagel- und Fußpilz werden durch Hefepilze verursacht, Infektionen durch anthropophile Erreger, wie beispielsweise Trichophyton rubrum, verlaufen in der Regel weniger heftig, dafür aber chronischer als Infektionen mit zoophilen Erregern, wie Trichophyton mentagrophytes. Diese Organismen sind durch direkten oder indirekten Kontakt mit infizierten Personen leicht übertragbar. Die Organismen sind gewöhnlich schwer zu eliminieren, da sie nicht nur die Oberfläche der Haut oder des Nagels besiedeln, sondern auch im subkutanen Gewebe vorhanden sind. Die meisten Fungizide, wie Clotrimazol, Terbinafin und Miconazol, weisen nur eine stark beschränkte Penetrationstiefe auf, was zu einem hohen Maß an Rückfällen führt. Die Wirksamkeit dieser Mittel ist deshalb recht eingeschränkt.

Das Bulbine-frutescens-Gel verstärkt jedoch in unerwarteter Weise den transdermalen Transport (auch bei Nägeln), wie durch Tests mit der Franz-Diffusionszelle nachgewiesen wurde. Außerdem kann der transdermale Transport durch die Verwendung eines Penetrationsverstärkers, wie etwa Polysorbat 80, ungesättigtes Phosphatidylcholin und ähnlichen Phospholipiden, Phytantriol und anderen Produkten, weiter verbessert werden, wie beispielsweise von Benson (Current Drug Delivery, 2, 23 (2005)) beschrieben.

Das Bulbine-frutescens-Gel konnte auch bei Kandidose erfolgreich eingesetzt werden. Der Hefepilz Candida albicans und verwandte Arten sind für verschiedene Arten von Kandidose verantwortlich, wie beispielsweise topische oder vaginale Kandidose. Die Behandlung mit herkömmlichen pharmazeutischen Produkten führt zu einer hohen Rückfallrate, insbesondere bei immungeschwächten Personen. Der Candida albicans kann mit Bulbine-frutescens-Gel wirksam behandelt werden.

Gegenstand der vorliegenden Erfindung ist daher die Verwendung des Bulbine-frutescens-Gels zur Herstellung eines Arzneimittels zur Behandlung von Hautoder Schleimhauterkrankungen, insbesondere Infektionen oder Entzündungen der Haut oder Schleimhaut, wie Pilzerkrankungen, oder zur Behandlung oder Vorbeugung von Verletzungen der Haut oder Schleimhaut, wie (Brand- )Wunden.

Der Ausdruck "Bulbine-frutescens-Gel" oder "Gel" wie in der vorliegenden Erfindung verwendet, bedeutet nicht zwangsläufig, dass die Darreichungsform oder Formulierung per se ein Gel darstellt. Vielmehr soll dies im Rahmen der Erfindung so verstanden werden, dass eine beliebige Darreichungsform oder Formulierung gewählt werden kann, die das "Gel" oder ein hieraus entsprechend weiterverarbeitetes Produkt als Wirkbestandteil enthält.

Weiterhin hat sich gezeigt, dass das Bulbine-frutescens-Gel auch in vorteilhafter Weise für die Körperpflege, beispielsweise als Antitranspirant, als hoch wirksamer Bestandteil eingesetzt werden kann. Direkt nach dem Ausscheiden ist der menschliche Schweiß geruchlos. Im Schweiß vorhandene Sterolester unterliegen jedoch einer mikrobiologischen Hydrolyse, wobei neben kurzkettigen Carbonsäuren (Essigsäure, Baldriansäure, Buttersäure und Isobuttersäure) Androstene gebildet werden. Androstene und kurzkettige Carbonsäuren sind verantwortlich für den wahrnehmbaren unerwünschten Geruch. Die Organismen, die für die Hydrolyse der Androstenester verantwortlich sind, werden oft der Familie der Lactobacillaceae, Milchsäure erzeugenden Organismen, zugeordnet, können aber auch andere gram-positive oder gram-negative Bakterien sein, wie etwa Staphylococcus epidermides. Durch Verringerung oder Beseitigung dieser Organismen kann der unangenehme Geruch vermindert oder vollständig beseitigt werden. Übliche Antitranspirant-wirkende Formulierungen, die zusätzlich das erfindungsgemäße Bulbine-frutescens-Gel enthalten, zeigen eine besonders hohe Wirksamkeit. Die Wirksamkeit derartiger Formulierungen wird durch den Aufbau der Hautbarriere weiter verbessert.

Ein weiterer Anwendungsbereich des erfindungsgemäßen Bulbine-fructescens-Gels ist bei der Mundpflege. In jüngerer Zeit hat sich gezeigt, dass eine gestörte orale Mikroflora Herz-Kreislauferkrankungen und Fehlgeburten verursachen kann. Das Bulbine-frutescens-Gel leistet bei der Regulierung der oralen Mikroflora einen äußerst wirksamen Beitrag, wobei der Mundgeruch als Gradmesser dienen kann. In einigen Fällen tragen Schwefelwasserstoff, Methylmercaptan und Dimethylsulfid neben verschiedenen alkylierten Aminen wesentlich zum Mundgeruch bei. In Versuchen wurde gezeigt, dass durch Ausspülen der Mundhöhle mit einer 10%igen wässerigen Lösung des Bulbine-frutescens-Gels unerwünschter Mundgeruch vollständig eliminiert werden konnte. Kleine Infektionen und Entzündungen des Zahnfleisches und der Mundschleimhaut verschwinden. Das Bulbine-frutescens-Gel ist damit herkömmlichen pharmazeutischen Präparaten, einschließlich der auf Chlorhexidindiglukonat und Wasserstoffperoxid basierenden Präparate zur Munddesinfektion, überlegen. S. salivarius wird vom Bulbine-frutescens-Gel kaum beeinträchtigt; demgegenüber sind Chlorhexidin und Wasserstoffperoxid für die kommensale Mikroflora in der Mundhöhle stark schädigend.

Es wurde bereits beschrieben, dass das Bulbine-frutescens-Gel das Immunsystem stärkt, was wahrscheinlich hauptsächlich auf die vorhandenen Polysaccharide zurückzuführen ist. Eine weitere Anwendung, die wahrscheinlich hiermit in Zusammenhang steht, ist die als Anti-Ageing-Produkt. Hierbei kommt es darauf an, dass ein positiver Einfluss auf beispielsweise die Hautelastizität, die Hautfestigkeit, den Wasserhaushalt der Haut sowie die Zelldifferenzierung und - proliferation vorliegt. Die Versuche zeigten nun, dass das Bulbine-frutescens-Gel, beispielsweise formuliert als Creme, zu einer deutlich verbesserten Elastizität und einer signifikant verbesserten Festigkeit der Haut führte, wobei gleichzeitig der Wasserverlust des subkutanen Hautgewebes stark verringert wurde. Es wurde auch ein deutlich verbessertes Hautbild beobachtet.

Die vorliegende Erfindung betrifft auch eine kosmetische oder pharmazeutische Zusammensetzung, umfassend das Bulbine-frutescens-Gel gemäß der vorliegenden Erfindung, in Mischung mit einem oder mehreren pharmazeutisch oder kosmetisch akzeptablen Trägermedien oder Hilfsstoffen. Pharmazeutisch oder kosmetisch akzeptable Träger und Hilfsstoffe sind dem Fachmann im Stand der Technik bekannt. Diese Träger und Hilfsstoffe umfassen beispielsweise lonen-Austauscher, Emulgatoren, Bindemittel, Suspendierhilfsmittel, Stabilisatoren, bakteriostatische Mittel, Antioxidantien, Lecithin, Serumproteine, Puffersubstanzen, pH-Einstellmittel, Konservierungsmittel, Wasser, Salze oder Elektrolyte, Substanzen auf Cellulosebasis und zahlreiche andere kosmetisch oder pharmazeutisch akzeptable Additive.

Zur therapeutischen oder kosmetischen Anwendung kann das Gel in irgendeiner herkömmlichen Dosierungsform in irgendeiner herkömmlichen Art und Weise auf der Haut oder Schleimhaut verabreicht werden. Bevorzugte Verabreichungswege sind transdermal und topisch. Die Dosierung des Bulbine-frutescens-Gels hängt u.a. ab von der Verabreichungsform, dem Verwendungszweck, der zu behandelnden Beeinträchtigung, Störung oder Erkrankung, dem Verwender, und den speziellen ausgewählten Trägern und/oder Hilfsstoffe. Erfindungsgemäß wird das Gel in der gewählten Darreichungsform bzw. Formulierung insbesondere durch äußere, lokale Auftragung verabreicht. Die Darreichungsform bzw. Formulierungen sind daher für eine transdermale oder topische Verabreichung ausgewählt und angepasst. Derartige Formulierungen werden in einer flüssigen, halbfesten oder festen Dosierungsform bereitgestellt und können beispielsweise in Form einer Lösung, beispielsweise als Tropfen, Tinktur, Öl oder Spüllösung, Suspension, Emulsion, Creme, Lotion/Milch, Schüttelmixtur, Puder, Paste, Spray, in transdermalen Pflastern oder dergleichen vorliegen.

Die Vorteile der vorliegenden Erfindung sind außerordentlich vielschichtig:
So wird das erfindungsgemäße Bulbine-frutescens-Gel durch ein speziell entwickeltes Verfahren aus den Blättern der Bulbine frutescens gewonnen, einer Wasserstoffperoxid-Behandlung unterzogen, das restliche Wasserstoffperoxid beseitigt und unter Verwendung eines geeigneten Stabilisierungsmittels ein Gel erhalten. Das erhaltene Gel kann so wie es ist formuliert werden oder es wird einer entsprechenden Weiterverarbeitung unterzogen und dann in eine geeignete Formulierungsform einbezogen.

Das erfindungsgemäße Gel kann sowohl im kosmetischen als auch therapeutischen Bereich Verwendung finden. Die Verabreichungsform ist hierbei nicht besonders beschränkt, wobei das Gel in der gewählten Formulierung zweckmäßiger Weise äußerlich auf der zu behandelnden Stelle örtlich aufgetragen wird. Der bevorzugte Verabreichungsweg ist transdermal oder topisch, wobei die das Gel enthaltende Formulierung bevorzugt lokal auf die Haut- oder Schleimhaut aufgetragen wird.

In völlig unerwarteter Weise konnte festgestellt werden, dass das erfindungsgemäße Bulbine-frutescens-Gel über antimikrobielle Eigenschaften verfügt, so dass entsprechende Produkte bereitgestellt werden können. Das Bulbine-frutescens-Gel kann, angereichert mit einer kleinen Menge Wasserstoffperoxid (z.B. 0,05 Gew.-%, ausgedrückt als H₂O₂) sogar als Desinfektionsmittel eingesetzt werden. Ein derartiges Kombinations-Desinfektionsmittel wirkt sogar gegen Staphylococcus aureus, einschließlich Methicillin-resistenten Staphylococcus aureus (MRSA) und Clindamycin-, Erythromycin- und Ciprofloxacin-resistenten Staphylococcus aureus (CEC-SA).

Das erfindungsgemäße Bulbine-frutescens-Gel kann in vorteilhafter Weise zur therapeutischen Behandlung von Erkrankungen der Haut und Schleimhaut eingesetzt werden, insbesondere gegen Infektionen und Entzündungen der Haut und Schleimhaut, wie Pilzerkrankungen, beispielsweise Fuß- und Nagelpilz oder Kandidose, insbesondere topische oder vaginale Kandidose, Verletzungen der Haut und Schleimhaut, wie Wunden, insbesondere Brandwunden, und kann beispielsweise auch zur Behandlung von Furunkeln und Karbunkeln eingesetzt werden. Das erfindungsgemäße Gel ist auch vorbeugend wirksam, beispielsweise um Verletzungen und/oder Wunden der Haut oder Schleimhaut zu verhindern.

Das Gel kann auch zur Stärkung der Immunabwehr verabreicht werden. Dies spielt insbesondere bei Patienten mit einem geschwächten Immunsystem eine Rolle, wie beispielsweise bei Diabetes- oder AIDS-Patienten. Durch die Kombinationswirkung des Bulbine-frutescens-Gel, das neben einer heilenden Wirkung auch über immunstärkende Eigenschaften verfügt, wird das Bulbine-frutescens-Gel zur Vermeidung/Vorbeugung und schnellen Heilung besonders erfolgreich eingesetzt.

Infektionen und Entzündungen klingen innerhalb kurzer Zeit ab, Wunden verheilen deutlich schneller und eine Immunstärkung findet statt, so dass Rückfälle weitgehend ausgeschlossen werden können.

Die gute Wundheilung durch das Bulbine-frutescens-Gel kann dazu führen, dass auf die zusätzliche Verabreichung von Antibiotika verzichtet werden kann.

Im kosmetischen Bereich wird das erfindungsgemäße Gel insbesondere zur Körperpflege, Mundpflege, als Antitranspirant und als Anti-Ageing-Produkt verwendet.

Die Wirkung ist zum Teil handelsüblichen Produkten weit überlegen, wobei die natürliche Mikroflora der Haut und der Schleimhäute nicht geschädigt, sondern vielmehr intakt erhalten bleibt.

Insbesondere in den Anwendungen im kosmetischen Bereich, beispielsweise als Anti-Ageing-Produkt, zeigt sich der positive Einfluß des erfindungsgemäßen Gels auf die Haut und Schleimhaut. So steigt die Hautelastizität, die Hautfestigkeit nimmt zu, der Wasserhaushalt der Haut sowie die Zelldifferenzierung und - proliferation werden nachhaltig positiv beeinflusst. Außerdem wird das Erscheinungsbild der Haut deutlich verbessert.

Im nachfolgenden experimentellen Teil sind einige Versuche detailliert geschildert, in denen die therapeutische und kosmetische Wirksamkeit des erfindungsgemäßen Bulbine-frutescens-Gels beschrieben sind. Selbstverständlich soll die vorliegende Erfindung hierdurch nicht beschränkt werden.

### Versuche zur mikrobiologischen Untersuchung und Bewertung des erfindungsgemäßen Bulbine-frutescens-Gels

Die antimikrobiellen Eigenschaften des erfindungsgemäßen Bulbine-frutescens-Gels wurden untersucht. Hierzu wurde nach entsprechender Verarbeitung der Blätter, der Behandlung mit Wasserstoffperoxid, der Beseitigung des restlichen Wasserstoffperoxids und der Stabilisierung unter Verwendung eines geeigneten Stabilisierungsmittels auf einen geeigneten pH-Wert das Gel, wie bereits detailliert erläutert, gewonnen.

### Kinetischer Provokationstest 1

Am Bulbine-frutescens-Gel wurde ein kinetischer Provokationstest an verschiedenen Organismen durchgeführt. Der Test wurde gemäß dem Verfahren der American Society for Testing and Materials (ASTM), Verfahren E1174, durchgeführt. Der Test wurde in Anwesenheit von 0,1 Gew.-% Humanserum mit einer Inokulation von 10⁶ CFU/g durchgeführt. Nach sechs Stunden wurden die folgenden logarithmischen Reduktionen beobachtet:

| | |
|---|---|
| Clostridium perfringens: | nach sechs Stunden keine log-Reduktion. |
| Clostridium tetani: | nach sechs Stunden keine log-Reduktion. |
| Streptococcus pyogenes Gruppe A: | nach sechs Stunden eine 4-log-Reduktion. |
| Staphylococcus aureus: | nach sechs Stunden eine 6-log-Reduktion (völlige Abtötung nach 1 Stunde). |
| Escherischia coli O157H7: | nach sechs Stunden eine 6-log-Reduktion (völlige Abtötung nach 6 Stunden). |
| Salmonella enteritidis Serotyp Panama: | nach sechs Stunden eine 6-log-Reduktion (völlige Abtötung nach 6 Stunden). |
| Candida albicans: | nach sechs Stunden eine 1-log-Reduktion. |
| Trichophyton mentagrophytes: | nach sechs Stunden eine 2-log-Reduktion. |

Für eine Anzahl von Organismen waren die Ergebnisse überraschend gut und standen in Widerspruch zu den in der Literatur veröffentlichten Ergebnissen.

### Kinetischer Provokationstest 2

In einem zweiten Test wurde das Bulbine-frutescens-Gel einem weiteren kinetischen Provokationstest unterzogen, aber nun in Anwesenheit von 1 Gew.-% menschlicher Fäkalien. Dieser Test wurde ebenfalls gemäß dem Verfahren der American Society for Testing and Materials (ASTM), Verfahren E1174, mit verschiedenen Inokulationen durchgeführt. Es wurde eine Dauer von zwei (2) Stunden gewählt.

Escherichia, Klebsiella & Enterobacter Spezies (aerobe gram-negative Stäbchen; Entero-bacteriaceae): Inokulation 10⁵ CFU/g. Völlige Abtötung nach 2 Stunden.

Bacteroides & Fusobacterium Spezies (anaerobe gram-negative Stäbchen): Inokulation 10⁴ CFU/g. Völlige Abtötung nach 1 Stunde.

Staphylococcus & Streptococcus Spezies (aerobe gram-positive Kokken): Inokulation 10⁴ CFU/g. Völlige Abtötung nach 2 Stunden.

Peptococcus & Peptostreptococcus (anaerobe gram-positive Kokken): Inokulation 10⁴ CFU/g. Völlige Abtötung nach 1 Stunde.

Lactobacillus & Bifidobacterium (anaerobe gram-positive nicht sporenbildende Stäbchen): Inokulation 10⁴ CFU/g. Völlige Abtötung nach 1 Stunde.

Clostridium Spezies (anaerobe gram-positive sporenbildende Stäbchen): Inokulation 10² CFU/g. Nach 2 Stunden keine Abtötung. Nach 24 Stunden eine 40%ige Reduktion.

Candida albicans: Inokulation 10² CFU/g. Völlige Abtötung nach 1 Stunde.

Die Ergebnisse waren wieder äußerst überraschend, völlig unerwartet und standen in Widerspruch zu früheren, in der Literatur angegebenen Daten. Die Ergebnisse zeigen Vorteile im Hinblick auf besondere mikrobielle Anforderungen. Das Bulbine-frutescens-Gel kann daher als funktioneller Inhaltsstoff zum Einsatz kommen.

### Kinetischer Provokationstest 3

Ein (100 % reines) Bulbine-frutescens-Gel ohne weitere Zusätze wurde einem weiteren kinetischen Provokationstest an verschiedenen Organismen unterzogen. Auch dieser Test wurde gemäß dem Verfahren der American Society for Testing and Materials (ASTM) mit einer Inokulation von 10⁶ CFU/g durchgeführt. Nach 6 Stunden wurden die folgenden logarithmischen Reduktionen beobachtet:

| | |
|---|---|
| Staphylococcus aureus: | nach sechs Stunden eine 6-log-Reduktion (völ-lige Abtötung nach 1 Stunde). |
| Enterococcus faecium: | nach sechs Stunden eine 6-log-Reduktion (völ-lige Abtötung nach 1 Stunde). |
| Escherichia coli: | nach sechs Stunden eine 6-log-Reduktion (völ-lige Abtötung nach 1 Stunde). |
| Candida albicans: | nach sechs Stunden eine 6-log-Reduktion (völ-lige Abtötung nach 1 Stunde). |
| Trichophyton mentagrophytes: | nach sechs Stunden eine 6-log-Reduktion (völ-lige Abtötung nach 1 Stunde). |

### Versuche zu den immunstimulierenden Eigenschaften des erfindungsgemäßen Bulbine-frutescens-Gels

### Wundheilung bei immungeschwächten Personen

Es wurden Versuche durchgeführt, um das Bulbine-frutescens-Gel bei der Wundheilung immungeschwächter Personen, insbesondere bei Patienten mit Diabetes Typ II und schlechter Wundheilung an den unteren Extremitäten (Füßen) zu testen.

Hierzu wurden acht Freiwillige mit nicht heilenden Wunden von durchschnittlich 11 cm² ausgewählt. Vier Freiwilligen wurde ein Placebo verabreicht und vier Freiwilligen wurde Bulbine-frutescens-Gel auf die Wunden aufgetragen. Bei den Freiwilligen, die mit Bulbine-frutescens-Gel behandelt wurden, wurde eine deutliche Verbesserung beobachtet: die Wunden schlossen sich innerhalb von 3 bis 4 Tagen. Bei den Freiwilligen, die mit dem Placebopräparat behandelt wurden, konnte keinerlei Verbesserung festgestellt werden. Da die Wunden bereits seit einem langen Zeitraum bestanden, in einem Fall mehr als ein Jahr, konnte die Bildung einer verfärbten Narbe nicht vermieden werden.

Das Bulbine-frutescens-Gel kann bei der Wundheilung schlecht heilender Wunden, wie etwa bei Patienten mit Diabetes Typ II, erfolgreich eingesetzt werden.

### Vorbeugende Wirkung bei Brandwunden

Es wurden Versuche durchgeführt, um das Bulbine-frutescens-Gel bei Brandwunden zu testen, die bei Krebspatienten durch die Bestrahlung mit γ-Strahlen entstanden sind. Die Bestrahlung mit γ-Strahlen wird nach dem chirurgischen Eingriff oft präventiv angewandt, zum Beispiel nach einer partiellen oder kompletten Brustentfernung. Sechs Patientinnen mit Brustkrebs wurden gebeten, das Gel präventiv auf den Körperbereich aufzubringen, der mit γ-Strahlen bestrahlt werden sollte. Nach dem Bestrahlungsprogramm wurden keine sichtbaren Brandwunden beobachtet, während die Kontrollgruppe mittlere bis schwere Brandwunden bekam.

Das Bulbine-frutescens-Gel kann daher in vorteilhafter Weise eingesetzt werden, um die während der Bestrahlung mit γ-Strahlen entstehenden Brandwunden zu verhindern und/oder zu heilen.

### Versuche zur Anwendungen des erfindungsgemäßen Bulbine-frutescens-Gels in der Kosmetik und zur Hautbehandlung

### Anwendung des Bulbine-frutescens-Gels als Antitranspirant

Wie bereits beschrieben wurde nach entsprechender Verarbeitung der Blätter, der Behandlung mit Wasserstoffperoxid, der Beseitigung des restlichen Wasserstoffperoxids und der Stabilisierung unter Verwendung eines geeigneten Stabilisierungsmittels das erfindungsgemäße Gel gewonnen. Das Gel wurde dann in eine antitranspirantiv-wirkende Formulierung einbezogen. Ein Beispiel für eine derartige Formulierung ist nachfolgend angegeben:

| | |
|---|---|
| demineralisiertes Wasser | 82,7 Gew.-% |
| Stearylglucosid (Emulgator) | 4,2 Gew.-% |
| Bulbine-frutescens-Gel | 6,0 Gew.-% |
| PPG-15-Stearylether | 4,0 Gew.-% |
| Decyloleat | 1,5 Gew.-% |
| Glycerylcaprylat | 1,2 Gew.-% |
| Hydroxyethylcellulose | 0,4 Gew.-% |
| | |
| Gesamt | 100 Gew.-% |

Der pH-Wert der Formulierung lag bei 4,5 bis 6,0.

Die gewonnene Formulierung war leicht viskos und konnte beispielsweise mit einem Roll-on-System aufgetragen werden. In einem Versuch (nicht-optimiert) wurde eine Geruchskontrolle für die Dauer von 8 Stunden durchgeführt, die in Form von "Schnüffeltests" stattfand. Die Formulierung verblieb wegen der getroffenen Auswahl von Weichmachern auf der Haut. Selbstverständlich sind auch andere beliebige Verabreichungsformen möglich, mit denen die Formulierung auf die Haut aufgebracht werden kann, wie beispielsweise in Form einer Creme, Lotion, Spray, Aerosol oder in anderer Form.

Das Bulbine-frutescens-Gel war für den Achsel- und Genitalbereich besonders wirksam. Eine Wirksamkeit im Fall der Trimethylaminurie-Störung liegt jedoch nicht vor.

### Anwendung von erfindungsgemäßem Bulbine-frutescens-Gel zur Behandlung von Nagelpilz und Fußpilz

Das erfindungsgemäß hergestellte Gel wurde in eine Formulierung einbezogen. Es wurde die folgende Zusammensetzung getestet:

| | |
|---|---|
| demineralisiertes Wasser | 82,6 Gew.-% |
| Bulbine-frutescens-Gel | 6,0 Gew.-% |
| Pentylenglycol | 5,0 Gew.-% |
| ungesättigtes Phosphatidylcholin | 4,0 Gew.-% |
| Isononylisononanoat | 2,0 Gew.-% |
| Hydroxyethylcellulose | 0,4 Gew.-% |
| | |
| Gesamt | 100 Gew.-% |

Der pH-Wert der Formulierung lag bei 4,5 bis 6,0.

Die Zusammensetzung wurde auf die Füße von Trägern mit Fußpilz-Erkrankung aufgetragen (sechs Freiwillige). Es zeigte sich, dass die Hefepilze nach 35 Tagen vollständig eliminiert waren und nach drei Monaten kein Rückfall zu verzeichnen war. Die kommensale Mikroflora auf der Haut wurde durch die Formulierung nicht nachteilig beeinflusst.

Folglich wurde gezeigt, dass das Bulbine-frutescens-Gel bei der Behandlung von Nagel- und Fußpilz wirksam verwendet werden kann.

Anwendung von Bulbine-frutescens-Gel in Mundpflegeprodukten

Placebo-kontrollierte Versuche wurden durchgeführt, in denen sich zeigte, dass das Ausspülen der Mundhöhle zweimal täglich mit 10%iger wässriger Lösung des Bulbine-frutescens-Gels unerwünschten Mundgeruch vollständig eliminierte. Gleichzeitig wurde beobachtet, dass kleine Infektionen des Zahnfleisches, die z. B. durch Streptococcus mutans und/oder S. sanguis verursacht wurden, reguliert werden konnten. Entzündungen des Zahnfleisches und der Mundschleimhaut, die mit 10%iger wässriger Bulbine-frutescens-Gel-Lösung behandelt wurden, verschwanden für gewöhnlich in 1 bis 2 Tagen, womit sie herkömmliche pharmazeutische Präparate, einschließlich der auf Chlorhexidindiglukonat und Wasserstoffperoxid basierenden Präparate zur Munddesinfektion, übertrafen. S. salivarius wurde vom Bulbine-frutescens-Gel kaum beeinträchtigt. Demgegenüber sind Chlorhexidin und Wasserstoffperoxid für die kommensale Mikroflora in der Mundhöhle stark schädigend.

Das Bulbine-frutescens-Gel war für die Mundpflege in hohem Maße wirksam, es ist sogar handelsüblichen Produkten überlegen.

### Anwendung von Bulbine-frutescens-Gel in Anti-Ageing-Produkten

Eine Creme, enthaltend das erfindungsgemäße Gel, mit folgender Zusammensetzung wurde auf ihre Anti-Aging-Eigenschaften getestet:

| | |
|---|---|
| demineralisiertes Wasser | 69,0 Gew.-% |
| Ethyhexylstearat | 6,0 Gew.-% |
| Cetearylalkohol | 6,0 Gew.-% |
| Bulbine-frutescens-Gel | 6,0 Gew.-% |
| Pentylenglycol | 5,0 Gew.-% |
| Glycerin | 3,0 Gew.-% |
| Ceteareth-6 | 2,5 Gew.-% |
| Ceteareth-25 | 2,5 Gew.-% |
| | |
| Gesamt | 100 Gew.-% |

Der pH-Wert der Creme lag bei 5,0 bis 6,0.

Als deutliche Anti-Aging-Eigenschaften wurden die Hautelastizität, die Hautfestigkeit, die Werte der Corneometer-Messung und der trans-epidermale Wasserverlust (TEWL) sowie die Zelldifferenzierung und -proliferation herangezogen. Die Ausrüstung wurde von der Courage & Khazaka Electronic GmbH, Köln, Deutschland, bezogen. Es zeigte sich, dass eine zweimalige tägliche Anwendung der Creme zu einer um 40 % verbesserten Elastizität und zu einer um 24 % verbesserten (Zug-)Festigkeit der Haut führte, wobei der Wasserverlust des subkutanen Gewebes an die Umgebung im Verhältnis zum Standard stark verringert wurde.

Weitere Versuche zeigten, dass der Zellstoffwechsel um 15 % anstieg. Eine Sichtprüfung der Haut zeigte ein deutlich verbessertes Hautbild.

Anwendung von Bulbine-frutescens-Gel bei topischer Kandidose

Getestet wurde eine Creme, enthaltend

| | |
|---|---|
| demineralisiertes Wasser | 69,0 Gew.-% |
| Ethyhexylstearat | 6,0 Gew.-% |
| Cetearylalkohol | 6,0 Gew.-% |
| Bulbine-frutescens-Gel | 6,0 Gew.-% |
| Pentylenglycol | 5,0 Gew.-% |
| Glycerin | 3,0 Gew.-% |
| Ceteareth-6 | 2,5 Gew.-% |
| Ceteareth-25 | 2,5 Gew.-% |
| | |
| Gesamt | 100 Gew.-% |

auf ihre Wirkung gegen opportunistische Candida albicans auf der Haut von sechs Freiwilligen. Der pH-Wert der Creme lag bei 5,0 bis 6,0. Obwohl die topische Kandidose mit Antibiotika gewöhnlich schwer zu behandeln ist, zeigte sich, dass die Creme wirksam war, selbst nach der Behandlung anderer Erkrankungen mit Antibiotika, die dem Candida albicans oft die Chance gibt, opportunistisch zu werden. Die Behandlung mit der Creme führte für gewöhnlich nach einer 3- bis 7-tägigen Behandlung zum Verschwinden der roten Punkte auf der Haut.

Es wird somit erfindungsgemäß erstmals ein Bulbine-frutescens-Gel bereitgestellt, dessen Wirkstoff auf pflanzlicher Basis ist, das sowohl im therapeutischen als auch kosmetischen Bereich für die Anwendung auf der Haut und Schleimhaut zum Einsatz kommen kann, in einer Vielzahl von Darreichungsformen formuliert werden kann und ein breites Wirkungsspektrum bietet. Die Wirksamkeit ist zum Teil überraschend hoch, wobei handelsübliche Produkte übertroffen werden. Trotzdem liegt eine hohe Verträglichkeit bei der Verwendung vor, wobei gleichzeitig die Eigenschaften der Haut- und Schleimhaut positiv beeinflusst werden.

## Patentansprüche

1. Bulbine-frutescens-Gel, erhältlich durch die Schritte:
(1) Waschen der Blätter der Bulbine frutescens mit Wasser und gegebenenfalls Zerkleinern der Blätter;
(2) Auspressen der Blätter unter Erhalt eines gelartigen Safts;
(3) Behandeln des gelartigen Safts mit Wasserstoffperoxid unter Rühren, wobei pro kg Saft 0,5 bis 20 g Wasserstoffperoxid zugegeben werden;
(4) Zerstören des Wasserstoffperoxids durch Zugabe einer pharmazeutisch oder kosmetisch akzeptablen Säure unter Rühren bis der Peroxidwert unter 0,02 meqO/kg liegt;
(5) Einstellen des pH-Werts auf 3,0 bis 4,0 unter Zugabe einer pharmazeutisch oder kosmetisch akzeptablen Säure und
(6) Erhalt des Gels unter Rühren sobald alle Komponenten gelöst sind.

2. Gel nach Anspruch 1,
**dadurch gekennzeichnet,**
**dass** die pharmazeutisch oder kosmetisch akzeptable Säure ausgewählt wird aus einer organischen Säure, bevorzugt ausgewählt aus der Gruppe, bestehend aus Adipinsäure, Alginsäure, Ascorbinsäure, Isoascorbinsäure (Erythorbinsäure), Asparaginsäure, Benzolsulfonsäure, Bernsteinsäure, 2-Acetoxybenzoesäure, Camphersäure, Camphersulfonsäure, Zimtsäure, Citronensäure, insbesondere Citronensäure-Monohydrat, Digluconsäure, Ethansulfonsäure, Glutaminsäure, Glykolsäure, Heptansäure, Hexansäure, Fumarsäure, 2-Hydroxyethansulfonsäure (Isethionsäure), Milchsäure, Maleinsäure, Hydroxymaleinsäure, Äpfelsäure, Malonsäure, Mandelsäure, Pamoasäure, Pectinsäure, Phenylessigsäure, 3-Phenylpropionsäure, Picrinsäure, Pivalinsäure, Pyruvinsäure, Stearinsäure, Sulfanilsäure, Weinsäure, p-Toluolsulfonsäure oder Mischungen dieser.

3. Gel nach Anspruch 1 oder 2,
**dadurch gekennzeichnet,**
**dass** die pharmazeutisch und kosmetisch akzeptable Säure in Verfahrensschritt (4) ausgewählt wird aus Ascorbinsäure oder Isoascorbinsäure (Erythorbinsäure) und in Verfahrensschritt (5) Citronensäure-Monohydrat eingesetzt wird.

4. Gel nach Anspruch 1,
**dadurch gekennzeichnet,**
**dass** die Anwendungsdauer in Schritt (3) derart gewählt wird, dass keine Verfärbung resultiert, bevorzugt eine Anwendungsdauer von wenigen Minuten bis 1 Stunde, besonders bevorzugt von wenigen Minuten bis eine halbe Stunde vorliegt.

5. Gel nach mindestens einem der vorangehenden Ansprüche 1 bis 4,
**dadurch gekennzeichnet,**
**dass** ein Konservierungsmittel zugesetzt wird, ausgewählt aus der Gruppe, bestehend aus Benzoesäure, Sorbinsäure, Parabenen (Methyl-, Ethyl-, Propyl-, Butyl-, Isobutyl- oder Benzyl-Paraben), Ameisensäure, Essigsäure, Propionsäure, Salicylsäure, p-Anissäure, Lävulinsäure und deren wasserlöslichen Salzen, Glycerol-/Polyglycerolmonoestern von Caprylsäure, Caprinsäure-Laurinestern, Diolen, wie 1,2-Dihydroxypentan, 1,2-Dihydroxyhexan oder 1,2-Dihydroxyoctan, oder 1-(2-Ethylhexyl)-Glycerylether oder Mischungen dieser.

6. Verfahren zur Herstellung eines Bulbine-frutescens-Gels nach einem der vorangehenden Ansprüche 1 bis 5, umfassend die Schritte:
(1) Waschen der Blätter der Bulbine frutescens mit Wasser und gegebenenfalls Zerkleinern der Blätter;
(2) Auspressen der Blätter unter Erhalt eines gelartigen Safts;
(3) Behandeln des gelartigen Safts mit Wasserstoffperoxid unter Rühren, wobei pro kg Saft 0,5 bis 20 g Wasserstoffperoxid zugegeben werden;
(4) Zerstören des Wasserstoffperoxids durch Zugabe einer pharmazeutisch oder kosmetisch akzeptablen Säure unter Rühren bis der Peroxidwert unter 0,02 meqO/kg liegt;
(5) Einstellen des pH-Werts auf 3,0 bis 4,0 unter Zugabe einer pharmazeutisch oder kosmetisch akzeptablen Säure und
(6) Erhalt des Gels unter Rühren sobald alle Komponenten gelöst sind.

7. Verfahren nach Anspruch 6,
**dadurch gekennzeichnet,**
**dass** die pharmazeutisch oder kosmetisch akzeptable Säure ausgewählt wird aus einer organischen Säure, bevorzugt ausgewählt aus der Gruppe, bestehend aus Adipinsäure, Alginsäure, Ascorbinsäure, Isoascorbinsäure (Erythorbinsäure), Asparaginsäure, Benzolsulfonsäure, Bernsteinsäure, 2-Acetoxybenzoesäure, Camphersäure, Camphersulfonsäure, Zimtsäure, Citronensäure, insbesondere Citronensäure-Monohydrat, Digluconsäure, Ethansulfonsäure, Glutaminsäure, Glykolsäure, Heptansäure, Hexansäure, Fumarsäure, 2-Hydroxyethansulfonsäure (Isethionsäure), Milchsäure, Maleinsäure, Hydroxymaleinsäure, Äpfelsäure, Malonsäure, Mandelsäure, Pamoasäure, Pectinsäure, Phenylessigsäure, 3-Phenylpropionsäure, Picrinsäure, Pivalinsäure, Pyruvinsäure, Stearinsäure, Sulfanilsäure, Weinsäure, p-Toluolsulfonsäure oder Mischungen dieser.

8. Verfahren nach Anspruch 6 oder 7,
**dadurch gekennzeichnet,**
**dass** die pharmazeutisch oder kosmetisch akzeptable Säure in Verfahrensschritt (4) ausgewählt wird aus Ascorbinsäure oder Isoascorbinsäure (Erythorbinsäure) und in Verfahrensschritt (5) Citronensäure-Monohydrat eingesetzt wird.

9. Verfahren nach mindestens einem der vorangehenden Ansprüche 6 bis 8,
**dadurch gekennzeichnet,**
**dass** die Anwendungsdauer in Verfahrensschritt (3) derart gewählt wird, dass keine Verfärbung resultiert, bevorzugt eine Anwendungsdauer von wenigen Minuten bis 1 Stunde, besonders bevorzugt von wenigen Minuten bis eine halbe Stunde eingesetzt wird.

10. Verfahren nach mindestens einem der vorangehenden Ansprüche 6 bis 9,
**dadurch gekennzeichnet,**
**dass** das erhaltene Gel einer Weiterverarbeitung unterzogen wird, aus ein oder mehreren der nachfolgenden Verfahrensweisen, die in beliebiger Reihenfolge kombiniert werden können: Filtrieren, Wasserverdampfung unter vermindertem Druck / erhöhter Temperatur, azeotrope Destillation unter vermindertem Druck / erhöhter Temperatur unter Verwendung eines geeigneten Co-Lösemittels, Sprühtrocknen oder Gefriertrocknen.

11. Bulbine-frutescens-Gel nach mindestens einem der vorangehenden Ansprüche 1 bis 5 zur Verwendung im medizinischen Bereich.

12. Verwendung des Bulbine-frutescens-Gels nach mindestens einem der vorangehenden Ansprüche 1 bis 5 im kosmetischen Bereich

13. Bulbine-frutescens-Gel nach mindestens einem der vorangehenden Ansprüche 1 bis 5 zur Verwendung in der Behandlung von Haut- oder Schleimhauterkrankungen, insbesondere Infektionen oder Entzündungen der Haut oder Schleimhaut, wie Pilzerkrankungen, oder in der Behandlung oder Vorbeugung von Verletzungen der Haut oder Schleimhaut, insbesondere bei (Brand-)Wunden.

14. Bulbine-frutescens-Gel nach mindestens einem der Ansprüche 1 bis 5 zur Verwendung nach Anspruch 11, **gekennzeichnet durch** eine Verwendung als Desinfektionsmittel, insbesondere gegen Staphylococcus aureus, einschließlich Methicillin-resistenten Staphylococcus aureus (MRSA) und Clindamycin-, Erythromycin- und Ciprofloxacin-resistenten Staphylococcus aureus (CEC-SA), oder als Mittel zur Stärkung der Immunabwehr.

15. Verwendung des Bulbine-frutescens-Gels nach Anspruch 12 zur Körperpflege, Mundpflege, als Antitranspirant und als Anti-Ageing-Produkt.

16. Kosmetische oder pharmazeutische Zusammensetzung, umfassend das Bulbine-frutescens-Gel nach mindestens einem der vorangehenden Ansprüche 1 bis 5, in Mischung mit einem oder mehreren pharmazeutisch oder kosmetisch akzeptablen Trägermedien oder Hilfsstoffen.

## Claims

1. A Bulbine frutescens gel, obtainable by the steps of:
(1) washing the leaves of Bulbine frutescens with water, and optionally chopping the leaves;
(2) squeezing the leaves for obtaining a gel-like sap;
(3) treating the gel-like sap with hydrogen peroxide while stirring, wherein 0.5 to 20 g of hydrogen peroxide are added per each kg of sap;
(4) destroying the hydrogen peroxide by adding a pharmaceutically or cosmetically acceptable acid while stirring, until the peroxide value is below 0.02 meqO/kg;
(5) adjusting the pH to a value of 3.0 to 4.0 while adding a pharmaceutically or cosmetically acceptable acid, and
(6) obtaining the gel while stirring once all components are dissolved.

2. The gel according to claim 1,
**characterized in that**
the pharmaceutically or cosmetically acceptable acid is selected from an organic acid, preferably selected from the group consisting of adipic acid, alginic acid, ascorbic acid, isoascorbic acid (erythorbic acid), aspartic acid, benzenesulfonic acid, succinic acid, 2-acetoxybenzoic acid, camphoric acid, camphorsulfonic acid, cinnamic acid, citric acid, in particular citric acid monohydrate, digluconic acid, ethanesulfonic acid, glutamic acid, glycolic acid, heptanoic acid, hexanoic acid, fumaric acid, 2-hydroxyethanesulfonic acid (isethionic acid), lactic acid, maleic acid, hydroxymaleic acid, malic acid, malonic acid, mandelic acid, pamoic acid, pectinic acid, phenylacetic acid, 3-phenylpropionic acid, picric acid, pivalic acid, pyruvic acid, stearic acid, sulfanilic acid, tartaric acid, p-toluenesulfonic acid, or mixtures of these.

3. The gel according to claim 1 or 2,
**characterized in that**
the pharmaceutically and cosmetically acceptable acid is selected from ascorbic acid or isoascorbic acid (erythorbic acid) in step (4) and citric acid monohydrate is used in step (5).

4. The gel according to claim 1,
**characterized in that**
the duration of the application is selected in step (3) in such a way that no discoloration is caused, preferably a duration of a few minutes to 1 hour, more preferably from a few minutes to half an hour.

5. The gel according to at least one of the preceding claims 1 to 4,
**characterized in that**
a preservative is added, selected from the group consisting of benzoic acid, sorbic acid, parabens (methyl, ethyl, propyl, butyl, isobutyl or benzyl paraben), formic acid, acetic acid, propionic acid, salicylic acid, p-anisic acid, levulinic acid and its water-soluble salts, glycerol / polyglycerol monoester of caprylic acid, lauric ester of capric acid, diols such as 1,2-dihydroxypentane, 1,2-dihydroxy hexane or 1,2-dihydroxyoctane, or 1-(2-ethylhexyl) glyceryl ether or mixtures of these.

6. A method for producing a Bulbine frutescens gel according to any one of the preceding claims 1 to 5, comprising the steps of:
(1) washing the leaves of Bulbine frutescens with water, and optionally chopping the leaves;
(2) squeezing the leaves and obtaining a gel-like sap;
(3) treating the gel-like sap with hydrogen peroxide while stirring, at which 0.5 to 20 g of hydrogen peroxide are added per each kg of sap;
(4) destroying the hydrogen peroxide by adding a pharmaceutically or cosmetically acceptable acid while stirring, until the peroxide value is below 0.02 meqO/kg;
(5) adjusting the pH to a value of 3.0 to 4.0 while adding a pharmaceutically or cosmetically acceptable acid, and
(6) obtaining the gel while stirring once all components are dissolved.

7. The method according to claim 6,
**characterized in that**
the pharmaceutically or cosmetically acceptable acid is selected from an organic acid, preferably selected from the group consisting of adipic acid, alginic acid, ascorbic acid, isoascorbic acid (erythorbic acid), aspartic acid, benzenesulfonic acid, succinic acid, 2-acetoxybenzoic acid, camphoric acid, camphorsulfonic acid, cinnamic acid, citric acid, in particular citric acid monohydrate, digluconic acid, ethanesulfonic acid, glutamic acid, glycolic acid, heptanoic acid, hexanoic acid, fumaric acid, 2-hydroxyethanesulfonic acid (isethionic acid), lactic acid, maleic acid, hydroxymaleic acid, malic acid, malonic acid, mandelic acid, pamoic acid, pectinic acid, phenylacetic acid, 3-phenylpropionic acid, picric acid, pivalic acid, pyruvic acid, stearic acid, sulfanilic acid, tartaric acid, p-toluenesulfonic acid, or mixtures of these.

8. The method according to claim 6 or 7,
**characterized in that**
the pharmaceutically or cosmetically acceptable acid is selected from ascorbic acid or isoascorbic acid (erythorbic acid) in step (4) and citric acid monohydrate is used in step (5).

9. The method according to at least one of the preceding claims 6 to 8,
**characterized in that**
the duration of the application is selected in step (3) in such a way that no discoloration is caused, preferably a duration of a few minutes to 1 hour, more preferably from a few minutes to half an hour.

10. The method according to at least one of the preceding claims 6 to 9,
**characterized in that**
the obtained gel is subjected to a further processing, comprising one or more of the following procedures, which can be combined in any order: filtration, evaporation of water under reduced pressure / elevated temperature, azeotropic distillation under reduced pressure / elevated temperature using a suitable co-solvent, spray drying or freeze drying.

11. The Bulbine frutescens gel according to at least one of the preceding claims 1 to 5 for use in the medical area.

12. Use of the Bulbine frutescens gel according to at least one of the preceding claims 1 to 5 in the cosmetic area.

13. The Bulbine frutescens gel according to at least one of the preceding claims 1 to 5 for use in the treatment of skin or mucosal diseases, in particular infections or inflammations of the skin or mucosa, such as fungal diseases, or in the treatment or prevention of lesions of the skin or mucosa, in particular in the case of (burn) wounds.

14. The Bulbine frutescens gel according to at least one of the preceding claims 1 to 5 for the use according to claim 11, **characterized in that** it is used as a disinfectant, in particular against Staphylococcus aureus, including methicillin-resistant Staphylococcus aureus (MRSA) and clindamycin, erythromycin and ciprofloxacin-resistant Staphylococcus aureus (SA-CEC), or as a means of strengthening the immune defense system.

15. The use of Bulbine frutescens gel according to claim 12 for body care, oral care, as an antiperspirant and as an anti-aging product.

16. Cosmetic or pharmaceutical composition comprising the Bulbine frutescens gel according to at least one of the preceding claims 1 to 5, in mixture with one or more pharmaceutically or cosmetically acceptable carrier media or excipients.

## Revendications

1. Gel de bulbine frutescens, pouvant être obtenu par les étapes :
(1) lavage des feuilles de la bulbine frutescens avec de l'eau et éventuellement fragmentation des feuilles ;
(2) expression des feuilles avec obtention d'un jus à consistance de gel ;
(3) traitement du jus à consistance de gel par du peroxyde d'hydrogène sous agitation, en ajoutant 0,5 à 20 g de peroxyde d'hydrogène par kg de jus ;
(4) destruction du peroxyde d'hydrogène par addition d'un acide pharmaceutiquement ou cosmétiquement acceptable, sous agitation, jusqu'à ce que l'indice de peroxyde soit inférieur à 0,02 mEq O/kg ;
(5) ajustement du pH à une valeur de 3,0 à 4,0 par addition d'un acide pharmaceutiquement ou cosmétiquement acceptable et
(6) obtention du gel sous agitation dès que tous les composants sont dissous.

2. Gel selon la revendication 1,
**caractérisé en ce que**
l'acide pharmaceutiquement ou cosmétiquement acceptable est choisi parmi un acide organique, de préférence est choisi dans le groupe constitué par l'acide adipique, l'acide alginique, l'acide ascorbique, l'acide iso-ascorbique (acide érythorbique), l'acide aspartique, l'acide benzénesulfonique, l'acide succinique, l'acide 2-acétoxybenzoïque, l'acide camphorique, l'acide camphosulfonique, l'acide cinnamique, l'acide citrique, en particulier l'acide citrique monohydraté, l'acide digluconique, l'acide éthanesulfonique, l'acide glutamique, l'acide glycolique, l'acide heptanoïque, l'acide hexanoïque, l'acide fumarique, l'acide 2-hydroxyéthanesulfonique (acide iséthionique), l'acide lactique, l'acide maléique, l'acide hydroxymaléique, l'acide malique, l'acide malonique, l'acide mandélique, l'acide pamoïque, l'acide pectinique, l'acide phénylacétique, l'acide 3-phénylpropionique, l'acide picrique, l'acide pivalique, l'acide pyruvique, l'acide stéarique, l'acide sulfanilique, l'acide d-tartrique, l'acide p-toluènesulfonique ou des mélanges de ceux-ci.

3. Gel selon la revendication 1 ou 2,
**caractérisé en ce que**
dans l'étape (4) du procédé l'acide pharmaceutiquement et cosmétiquement acceptable est choisi parmi l'acide ascorbique et l'acide iso-ascorbique (acide érythorbique) et dans l'étape (5) du procédé on utilise l'acide citrique monohydraté.

4. Gel selon la revendication 1,
**caractérisé en ce que**
la durée d'application dans l'étape (3) est choisie de manière qu'il n'en résulte aucun changement de couleur, de préférence est présente une durée d'application de quelques minutes à 1 heure, de façon particulièrement préférée de quelques minutes à une demi-heure.

5. Gel selon au moins l'une quelconque des revendications 1 à 4 précédentes,
**caractérisé en ce**
**qu'**on ajoute un conservateur, choisi dans le groupe constitué par l'acide benzoïque, l'acide sorbique, les parabènes (méthyl-, éthyl-, propyl-, butyl-, isobutyl- ou benzyl-parabène), l'acide formique, l'acide acétique, l'acide propionique, l'acide salicylique, l'acide p-anisique, l'acide lévulinique et leurs sels hydrosolubles, des monoesters de glycérol/polyglycérol avec l'acide caprylique, des esters lauryliques d'acide caprique, des diols, tels que le 1,2-dihydroxypentane, le 1,2-dihydroxyhexane ou le 1,2-dihydroxy-octane, ou le 1-(2-éthylhexyl)-glycéryléther ou des mélanges de ceux-ci.

6. Procédé pour la préparation d'un gel de bulbine frutescens selon l'une quelconque des revendications 1 à 5 précédentes, comprenant les étapes :
(1) lavage des feuilles de la bulbine frutescens avec de l'eau et éventuellement fragmentation des feuilles ;
(2) expression des feuilles avec obtention d'un jus à consistance de gel ;
(3) traitement du jus à consistance de gel par du peroxyde d'hydrogène sous agitation, en ajoutant 0,5 à 20 g de peroxyde d'hydrogène par kg de jus ;
(4) destruction du peroxyde d'hydrogène par addition d'un acide pharmaceutiquement ou cosmétiquement acceptable, sous agitation, jusqu'à ce que l'indice de peroxyde soit inférieur à 0,02 mEq O/kg ;
(5) ajustement du pH à une valeur de 3,0 à 4,0 par addition d'un acide pharmaceutiquement ou cosmétiquement acceptable et
(6) obtention du gel sous agitation dès que tous les composants sont dissous.

7. Procédé selon la revendication 6,
**caractérisé en ce que**
l'acide pharmaceutiquement ou cosmétiquement acceptable est choisi parmi un acide organique, de préférence est choisi dans le groupe constitué par l'acide adipique, l'acide alginique, l'acide ascorbique, l'acide iso-ascorbique (acide érythorbique), l'acide aspartique, l'acide benzénesulfonique, l'acide succinique, l'acide 2-acétoxybenzoïque, l'acide camphorique, l'acide camphosulfonique, l'acide cinnamique, l'acide citrique, en particulier l'acide citrique monohydraté, l'acide digluconique, l'acide éthanesulfonique, l'acide glutamique, l'acide glycolique, l'acide heptanoïque, l'acide hexanoïque, l'acide fumarique, l'acide 2-hydroxyéthanesulfonique (acide iséthionique), l'acide lactique, l'acide maléique, l'acide hydroxymaléique, l'acide malique, l'acide malonique, l'acide mandélique, l'acide pamoïque, l'acide pectinique, l'acide phénylacétique, l'acide 3-phénylpropionique, l'acide picrique, l'acide pivalique, l'acide pyruvique, l'acide stéarique, l'acide sulfanilique, l'acide d-tartrique, l'acide p-toluènesulfonique ou des mélanges de ceux-ci.

8. Procédé selon la revendication 6 ou 7,
**caractérisé en ce que**
dans l'étape (4) du procédé l'acide pharmaceutiquement ou cosmétiquement acceptable est choisi parmi l'acide ascorbique et l'acide iso-ascorbique (acide érythorbique) et dans l'étape (5) du procédé on utilise l'acide citrique monohydraté.

9. Procédé selon au moins l'une quelconque des revendications 6 à 8 précédentes,
**caractérisé en ce que**
la durée d'application dans l'étape (3) est choisie de manière qu'il n'en résulte aucun changement de couleur, de préférence on utilise une durée d'application de quelques minutes à 1 heure, de façon particulièrement préférée de quelques minutes à une demi-heure.

10. Procédé selon au moins l'une quelconque des revendications 6 à 9 précédentes,
**caractérisé en ce**
**qu'**on soumet le gel obtenu à un traitement ultérieur, choisi parmi une ou plusieurs des opérations suivantes, qui peuvent être combinées de façon quelconque : filtration, évaporation de l'eau sous pression réduite/à température élevée, distillation azéotropique sous pression réduite/à température élevée avec utilisation d'un co-solvant approprié, séchage par atomisation ou lyophilisation.

11. Gel de bulbine frutescens selon au moins l'une quelconque des revendications 1 à 5 précédentes, pour utilisation dans le domaine médical.

12. Utilisation du gel de bulbine frutescens selon au moins l'une quelconque des revendications 1 à 5 précédentes, dans le domaine cosmétique.

13. Gel de bulbine frutescens selon au moins l'une quelconque des revendications 1 à 5 précédentes, pour utilisation dans le traitement de maladies de peau ou des muqueuses, en particulier d'infections ou d'inflammations de la peau ou de la muqueuse, telles que des mycoses, ou dans le traitement ou la prévention de lésions de la peau ou de la muqueuse, en particulier pour des plaies (dues à des brûlures).

14. Gel de bulbine frutescens selon au moins l'une quelconque des revendications 1 à 5 précédentes, pour utilisation selon la revendication 11, **caractérisé par** une utilisation en tant que désinfectant, en particulier contre *Staphylococcus aureus,* y compris *Staphylococcus aureus* résistant à la méthicilline (MRSA) et *Staphylococcus aureus* résistant à la clindamycine, l'érythromycine et la ciprofloxacine (CEC-SA), ou en tant qu'agent destiné au renforcement de la défense immunitaire.

15. Utilisation du gel de bulbine frutescens selon la revendication 12, pour le soin du corps, le soin buccal, comme antisudoral et comme produit anti-âge.

16. Composition cosmétique ou pharmaceutique, comprenant le gel de bulbine frutescens selon au moins l'une quelconque des revendications 1 à 5 précédentes, en mélange avec un ou plusieurs milieux véhicules ou adjuvants pharmaceutiquement ou cosmétiquement acceptables.
